# EUROPEAN PATENT APPLICATION

(11) **EP 3 970 732 A1**
(43) Date of publication of application: **23.03.2022**
(21) Application number: 21151586.1
(22) Date of filing: 14.01.2021
(51) Int. Cl.: A61K 35/28, A61P 31/12

(54) **USE OF MESENCHYMAL STEM CELLS IN THE TREATMENT OF VIRAL INFECTIONS AND/OR COMPLICATIONS CAUSED BY VIRAL INFECTIONS**

(30) Priority: 17.09.2020 CN 202010979148; 17.09.2020 CN 202010979140; 17.09.2020 CN 202010979138
(71) Applicant: Hunan Yuanpin Cell Technology Co. Ltd, Changsha City, Hunan (CN)
(72) Inventor: XUE, Zhigang, Changsha City, Hunan (CN); YI, Ning, Changsha City, Hunan (CN); QI, Lingbin, Changsha City, Hunan (CN); LV, Bo, Changsha City, Hunan (CN); LI, Chanyi, Changsha City, Hunan (CN); ZHENG, Chunbing, Changsha City, Hunan (CN); LI, Weilin, Changsha City, Hunan (CN); HUA, Jiangzhou, Changsha City, Hunan (CN); YAN, Tenglong, Changsha City, Hunan (CN); YANG, Yuan, Changsha City, Hunan (CN)
(74) Representative: Comoglio, Elena

(57) **Abstract**

The present disclosure provides the use of mesenchymal stem cells (MSC) in the treatment of viral infection and/or complications caused by the viral infection, which belongs to the technical field of biomedical engineering. MSC can highly express multiple antioxidative stress genes and secrete multiple antioxidative stress factors under the stimulation of oxidative stress, inhibit inflammatory reaction and excessive activation of immune cells, thus improving inflammatory indexes and myocardial injury indexes of human bodies. MSC can regulate the expression profile of monocyte genes and monocyte toxicity genes in patients with viral infection, inhibit the excessive immune response, protect alveolar function, and reduce the damage of lung and whole body organs in patients with viral infection. MSC can regulate the expression of antivirus-related genes and coagulation-related genes of terminally differentiated cell subsets in patients with viral infection, inhibit the excessive activation of monocytes in patients with viral infection, and effectively treat the complications of patients with viral infection.

## Description

### CROSS REFERENCE TO RELATED APPLICATIONS

This disclosure claims the priority of Chinese Patent Application NO. 202010979140.3 entitled "Use of MSC for regulating gene expression of terminally differentiated cells in patients with viral infection" , Application NO. 202010979138.6 entitled "Use of mesenchymal stem cells for improving oxidative stress state of human body" and Application NO. 202010979148.X entitled "Use of mesenchymal stem cells for regulating monocytes in patients with viral infection", all of which were filed with China National Intellectual Property Administration on September 17, 2020 and are incorporated herein by reference in its entirety.

### TECHNICAL FIELD

The present disclosure relates to the technical field of biomedical engineering, and particularly relates to the use of mesenchymal stem cells for regulating monocytes in patients with viral infection, regulating gene expression of terminally differentiated cells in patients with viral infection, or improving oxidative stress state of human body.

### BACKGROUND ART

Viral infection refers to infectious diseases caused by viruses that can parasitize and reproduce in human body and cause diseases. The main manifestations are fever, headache, general malaise and other systemic poisoning symptoms, as well as local symptoms which are caused by inflammatory damage caused by virus parasitism and invasion of tissues and organs. Patients with viral infection are prone to induce cytokine storms, produce inflammatory reactions, and have various complications. Moreover, viral infections (such as influenza, SARS, MERS and Ebola) spread rapidly among human beings, posing a great threat to human health. Particularly, novel coronavirus (COVID-19 virus) broke out all over the world in 2020, causing serious losses to human health and economy. The pathogen of COVID-19 has been confirmed to be a *novel coronavirus,* which is now called *Severe Acute Respiratory Syndrome Coronavirus 2* (SARS-CoV-2). At present, neither wonder drugs nor vaccines against the virus have been successfully developed, and the emergence of SARS-CoV-2 has brought a difficult treatment dilemma to clinicians. Most infected patients show nonspecific symptoms, such as fever, dry cough and fatigue. Most patients have a good prognosis, while some patients with severe cases can rapidly develop acute respiratory distress syndrome, septic shock, metabolic acidosis, coagulation disorders and even death. The deterioration of the patient's condition may be due to the cytokine storm in the body. In view of SARS-CoV-2 being a new strain causing a global pandemic, there is an urgent need for effective targeted therapy, including antiviral and immunotherapy.

At present, there is a lack of specific treatment for viral infection diseases and complications of viral infection, with support and symptomatic treatment as the main treatment, while antiviral drugs and hormone therapy are not effective treatment options. Usually, the patient's symptoms are relieved through auxiliary means, and it is dominated that the patients rely on their own immunity to overcome the viral infection and the complications. The number of CD4+ and CD8+ T cells in the peripheral blood of severe patients with viral infection often decreases significantly, and some patients have abnormal immune activation, resulting in cytokine storm syndrome (CSS), which in turn leads to serious damage of organs such as lungs and to death. The main countermeasure in the prior arts is to use glucocorticoids. However, due to the overactivation of immunity and rapid transformation of immunosuppression in patients with viral infection, the use of hormones may lead to the risk of delayed virus clearance and secondary infection.

At present, there are no reports and records on the use of mesenchymal stem cells in the treatment of viral infection and/or complications caused by viral infection.

### SUMMARY OF THE INVENTION

The objective of the disclosure is to provide the use of mesenchymal stem cells in the treatment of viral infection and/or complications caused by viral infection.

In order to achieve the above objective, the present disclosure provides the following technical schemes.

The present disclosure provides the use of mesenchymal stem cells for regulating monocytes in patients with viral infection, regulating gene expression of terminally differentiated cells in patients with viral infection, or improving oxidative stress state of human body.

In some embodiments, when the mesenchymal stem cells are used for regulating monocytes in patients with viral infection, the mesenchymal stem cells are used for regulating gene expression and/or toxic gene expression of monocytes in patients with viral infection to achieve effective treatment of a complication induced by viral infection;

when the mesenchymal stem cells are used for regulating the gene expression of terminally differentiated cells in patients with viral infection, the mesenchymal stem cells are used for regulating the expression of antivirus-related genes and/or coagulation-related genes of terminally differentiated cell subsets in patients with viral infection to achieve effective treatment of a complication induced by viral infection;

when the mesenchymal stem cells are used for improving oxidative stress state of human body, the mesenchymal stem cells are used for improving the oxidative stress state of patients with viral infection.

In some embodiments, the mesenchymal stem cells are used for improving the oxidative stress state of patients with viral infection by highly expressing multiple antioxidant stress genes and secreting multiple antioxidative stress factors.

In some embodiments, the antioxidative stress genes highly expressed in the mesenchymal stem cells include GPX4, GSTP1, PRDX1, PRDX2, PRDX3, PRDX4, PRDX5, PRDX6, SOD1, SOD2, TXNRD1, NQO1, PARK7, PSMB5, SLC38A1 and VIM.

In some embodiments, the antioxidative stress factors secreted by the mesenchymal stem cells under oxidative stress conditions include GSTP1, PRDX1, PRDX2, VIM, GPX4, PSMB5 and SOD1.

In some embodiments, the complication induced by viral infection is acute respiratory distress syndrome.

In some embodiments, when the mesenchymal stem cells are used for regulating monocytes in patients with viral infection, the use comprising steps of:
S1, preparing a mesenchymal stem cell preparation;
S2, screening and grouping patients with viral infection, at least comprising a mesenchymal stem cell treatment group and a conventional treatment group;
S3, carrying out conventional treatment on patients with viral infection in the conventional treatment group while incorporating the prepared mesenchymal stem cell preparation into the conventional treatment regimen of the conventional treatment group so as to impose a combined treatment on the patients with viral infection in the mesenchymal stem cell treatment group;
S4, sampling and analyzing the peripheral blood mononuclear cells of the healed patients by using the single cell sequencing technology to obtain the expression profile of monocyte genes and monocyte toxicity genes of the healed patients.

In some embodiments, when the mesenchymal stem cells are used for regulating gene expression of terminally differentiated cells in patients with viral infection, the use comprising steps of:
S1, preparing a mesenchymal stem cell preparation;
S2, screening and grouping patients with viral infection, at least comprising a mesenchymal stem cell treatment group and a conventional treatment group;
S3, carrying out conventional treatment on patients with viral infection in the conventional treatment group while incorporating the prepared mesenchymal stem cell preparation into the conventional treatment regimen of the conventional treatment group so as to impose a combined treatment on the patients with viral infection in the mesenchymal stem cell treatment group;
S4, sampling and analyzing peripheral blood mononuclear cells of the healed patients by using a single cell sequencing technology to obtain the expression profile of antivirus-related genes and coagulation-related genes of terminally differentiated cell subsets of the healed patients.

In some embodiments, the healed patients include patients with viral infection cured in mesenchymal stem cell treatment group and patients with viral infection cured in conventional treatment group.

In some embodiments, when the mesenchymal stem cells are used for improving the oxidative stress state of human body, the use comprising steps of:
S 1, preparing mesenchymal stem cell preparations;
S2, detecting the mesenchymal stem cells prepared in the step S1 by using a single cell sequencing technology, and analyzing the expression of antioxidative stress genes in the mesenchymal stem cells;
S3, respectively culturing the mesenchymal stem cells prepared in step S1 under different oxidative stress stimulation conditions, and detecting the secretion profile of antioxidative stress factors in the cultured mesenchymal stem cells.

In some embodiments, the specific implementation of step S2 includes: detecting the prepared mesenchymal stem cells by using a single cell sequencing technology, and analyzing the expression of multiple antioxidative stress genes of the mesenchymal stem cells.

In some embodiments, the specific implementation of step S3 includes: firstly, culturing the prepared mesenchymal stem cells under conventional conditions and oxidative stress stimulation conditions with different concentrations, and then detecting and analyzing the secretion profile of antioxidative stress factors in the mesenchymal stem cells under different conditions and in different periods.

In some embodiments, the preparation method of the mesenchymal stem cell preparation comprise:
S11, collecting an umbilical cord and placing the umbilical cord in a culture dish, and then cleaning the umbilical cord tissue with normal saline;
S12, cutting the cleaned umbilical cord tissue into small tissue pieces and planting the small tissue pieces in a culture dish so as to perform the culturing;
S13, removing the culture solution from the culture dish, washing with normal saline, adding pancreatin for digestion until the cells in the culture dish are digested, adding stopping solution to stop digestion, transferring the cell suspension to a centrifuge tube for centrifugation and discarding supernatant, resuspending the cells with an appropriate amount of culture solution and counting, and finally planting the cell suspension in a new culture dish so as to culture the cell suspension according to the counting result;
S14, removing the culture solution from the new culture dish in step S13, washing it with normal saline, adding pancreatin for digestion and adding stop solution to stop digestion until the cells in the culture dish are digested, filtering with a cell sieve, transferring the filtered cell suspension to a centrifuge tube, counting and centrifuging to discard supernatant, preparing a cell preparation suspension, adding the cell preparation suspension to resuspend the cells, and finally transferring the cell suspension to a transfer bag and placing the transfer bag in a low temperature environment for subsequent withdrawal, thus completing the preparation of mesenchymal stem cell preparation;
S15, qualifying the mesenchymal stem cell preparation prepared in step S14.

The present disclosure also provides a method for regulating the gene expression of terminal differentiation cells and monocyte genes of patients with viral infection or improving the oxidative stress state of human body, which comprises the following steps:
1) preparing a mesenchymal stem cell preparation;
2) incorporating mesenchymal stem cell preparation into conventional treatment regimen for combined treatment of patients with viral infection.

In some embodiments, the method for combined treatment of patients with viral infection by using the mesenchymal stem cell preparation comprises a treatment by giving the mesenchymal stem cell preparation by means of peripheral intravenous infusion.

In some embodiments, the infusion dose of the mesenchymal stem cell preparation is (0.8-1) × 10⁶ cells per kilogram of body weight.

In some embodiments, the peripheral intravenous infusions of the mesenchymal stem cell preparation is performed for 3 times, with an interval of 3 days between every two infusions.

Compared with the prior arts, the present disclosure provides the use of mesenchymal stem cells in the treatment of viral infection and/or complications caused by viral infection. According to the present disclosure, the mesenchymal stem cells can highly express multiple antioxidative stress genes and secrete multiple antioxidative stress factors under the stimulation of oxidative stress, and can effectively inhibit the inflammatory reaction of human bodies and the excessive activation of immune cells through multiple antioxidative stress factors, so that the inflammatory indexes and myocardial injury indexes of human bodies can be effectively improved. In the present disclosure, mesenchymal stem cells can achieve effective regulation of monocyte genes expression and monocyte toxicity genes expression in patients with viral infection, timely and effectively inhibit the excessive immune response in patients, and the alveolar function in the patient is effectively protect, at the same time, it greatly reduces the damage of lungs and whole body organs of patients with viral infection. Therefore, by using mesenchymal stem cells, an effective treatment of the complications in patients with viral infection can be achieved by expressing the monocyte genes and monocyte toxicity genes. In the present disclosure, the mesenchymal stem cells provides effective regulation of the expression of antivirus-related genes and coagulation-related genes of terminally differentiated cell subsets of patients with viral infection, thereby effectively inhibiting the excessive activation of monocytes of patients with viral infection and realizing effective treatment of complications of patients with viral infection.

### BRIEF DESCRIPTION OF THE DRAWINGS

FIG. 1 is a flow chart of the steps of using mesenchymal stem cells in regulating monocytes of patients with viral infection;
FIG. 2 is a flow chart of the preparation method of the mesenchymal stem cell preparation of the present disclosure;
FIG. 3 is a flow chart of the steps of using mesenchymal stem cells in improving the oxidative stress state of human body;
FIG. 4 is a schematic diagram of the expression level of antioxidative stress genes in mesenchymal stem cells according to the present disclosure;
FIG. 5 is a flow chart of steps of using MSC in regulating gene expression of terminally differentiated cells of patients with viral infection;
FIG. 6 is a dimension reduction analysis diagram of 20 subsets of peripheral blood mononuclear cells in the present disclosure;
FIG. 7 is a schematic diagram of two monocyte subsets (mono4 and mono5) of patients with viral infection complications in MSC treatment group, conventional treatment group and healthy group of the present disclosure;
FIG. 8 is a schematic diagram of the proportion of different genes in CD14+ cell subsets of MSC treatment group, conventional treatment group and healthy group in the present disclosure.

### DETAILED DESCRIPTION OF THE EMBODIMENTS

In order to enable those skilled in the art to better understand the technical scheme of the present disclosure, the present disclosure will be further described in detail with reference to the accompanying drawings.

It should be noted that the original treatment regimen mentioned in the present disclosure refers to the treatment regimen adopted in the diagnosis and treatment regimen of novel coronavirus by the Office of the National Health Commission and Office of the State Administration of Traditional Chinese Medicine, "MSC+" represents the MSC treatment group, "MSC-" represents the conventional treatment group, and "Healthy" represents the healthy group, wherein MSC (Mesenchymal Stem Cell) represents mesenchymal stem cells.

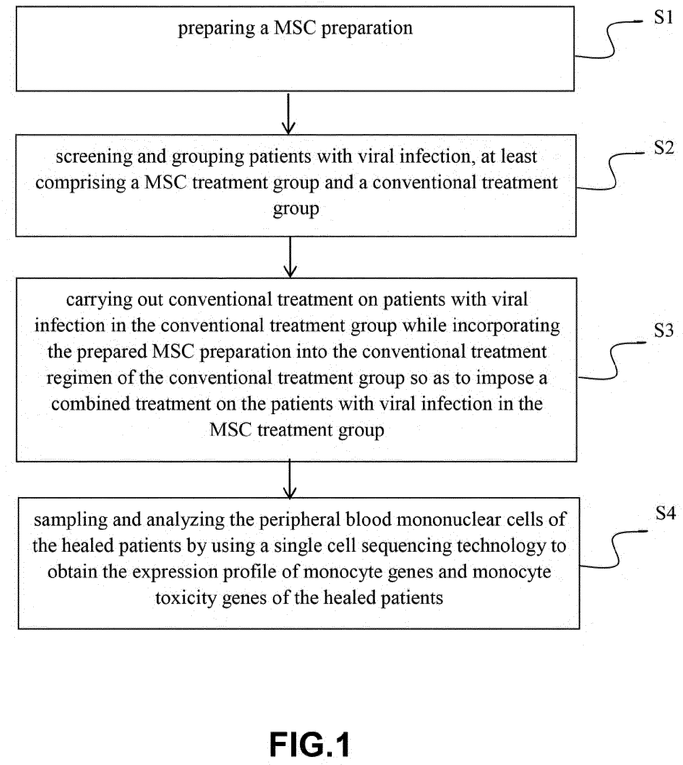

As shown in FIG. 1, the present disclosure provides the use of mesenchymal stem cells (MSC) in regulating monocytes of patients with viral infection, and realizes effective treatment of a complication induced by viral infection by using MSC to regulate gene expression of monocytes and monocyte toxicity gene. Wherein, the complication induced by viral infection is acute respiratory distress syndrome, and the use comprising steps of:
S 1, preparing a mesenchymal stem cell preparation;
S2, screening and grouping patients with viral infection, at least comprising a MSC treatment group and a conventional treatment group;
S3, carrying out conventional treatment on patients with viral infection in the conventional treatment group while incorporating the prepared mesenchymal stem cell preparation into the conventional treatment regimen of the conventional treatment group so as to impose a combined treatment on the patients with viral infection in the MSC treatment group;
S4, sampling and analyzing the peripheral blood mononuclear cells of the healed patients by using the single cell sequencing technology to obtain the expression profile of monocyte genes and monocyte toxicity genes of the healed patients.

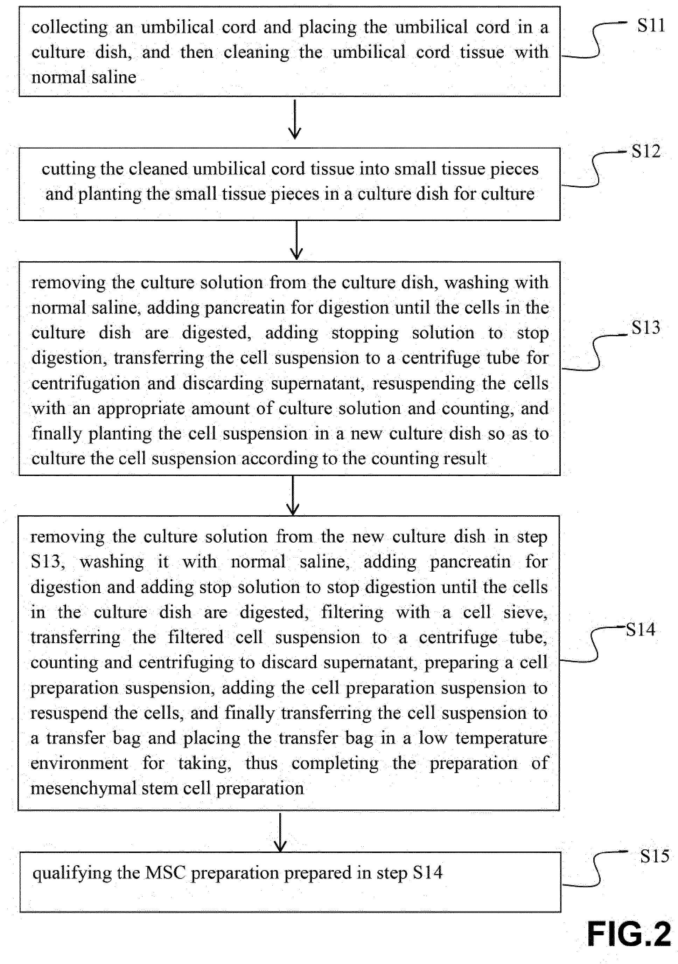

As shown in FIG. 2, the preparation method of the mesenchymal stem cell preparation comprises:
S11, collecting an umbilical cord and placing the umbilical cord in a culture dish, and then cleaning the umbilical cord tissue with normal saline;
S12, cutting the cleaned umbilical cord tissue into small tissue pieces and planting the small tissue pieces in a culture dish so as to perform the culturing;
S13, removing the culture solution from the culture dish, washing with normal saline, adding pancreatin for digestion until the cells in the culture dish are digested, adding stopping solution to stop digestion, transferring the cell suspension to a centrifuge tube for centrifugation and discarding supernatant, resuspending the cells with an appropriate amount of culture solution and counting, and finally planting the cell suspensions in a new culture dish so as to culture the cell suspension according to the counting result;
S14, removing the culture solution from the new culture dish in step S13, washing it with normal saline, adding pancreatin for digestion and adding stop solution to stop digestion until the cells in the culture dish are digested, filtering with a cell sieve, transferring the filtered cell suspension to a centrifuge tube, counting and centrifuging to discard supernatant, preparing a cell preparation suspension, adding the cell preparation suspension to resuspend the cells, and finally transferring the cell suspension to a transfer bag and placing the transfer bag in a low temperature environment for subsequent withdrawal, thus completing the preparation of mesenchymal stem cell preparation;
S15, qualifying the mesenchymal stem cell preparation prepared in step S14.

In the example, the mesenchymal stem cell preparation are prepared by using human umbilical cord mesenchymal stem cells (hUC-MSC), and the preparation process is carried out in a sterilized clean bench, and the instruments and consumable supplies required in the preparation process need to be disinfected with 75% alcohol, the collected materials or cells from the culture dish and the specific operation process need to be marked in detail during the preparation process. At the same time, the stem cells can be frozen between step S13 and step S14. If it is necessary to perform step S14, the cryopreserved stem cells need to be resuscitated before preparing the stem cells preparation in step S14, the freezing and resuscitation operations of stem cells belong to the prior art, and they will not be described in detail here.

In the example, the quality detection of the mesenchymal stem cell preparation also includes collection quality detection, admittance detection of umbilical cord mesenchymal stem cells in the primary cell bank and admittance detection of umbilical cord mesenchymal stem cells in master cell bank, and the steps and standards for the quality detection are consistent with the existing common detection, and they will not be described in detail here.

The combined treatment refers to the treatment in which the prepared mesenchymal stem cell preparation are incorporated into the conventional treatment regimen of the conventional treatment group as the combined treatment regimen of the MSC treatment group, and the mesenchymal stem cell preparation are used as the only interference factor in the conventional treatment regimen to carry out combined medication for patients with viral infection, and the combined medication situation is tracked and recorded in the combined treatment regimen. In the example, the mesenchymal stem cell preparation are infused through the peripheral vein during the whole MSC treatment group, and the infusion dose of the mesenchymal stem cell preparation is preferably (0.8-1)×10⁶ cells per kilogram of body weight, the infusions is conducted for 3 times, and the interval between every two infusions is 3 days, the infusion rate is 10 drops/ min from 0 to 15min, and the infusion is continued at a rate of 20 drops/ min if there is no significant response.

In this example, the selected patients with normal novel coronal positive (i.e., patients with viral infection of SARS-CoV-2) are divided into two groups and use them as the samples of the MSC treatment group and the conventional treatment group (i.e., the original treatment plan). The peripheral blood mononuclear cells of the healed patients are sampled after the patients with viral infection in the both groups are cured. The peripheral blood mononuclear cells of the healed patients (that is, cell samples from patients with viral infectious complications) are analyzed in separate groups by using the single cell sequencing technology, and the analysis results are processed. Thus, the expression profile of monocyte genes and monocyte toxic genes of the healed patients are obtained, the details are as follows. Table 1 shows the relationship table of monocyte gene expression in healed patients between MSC treatment group and conventional treatment group. Table 2 shows the cytotoxic gene table highly expressed in CD14 cell subsets (mono3) in healed patients between MSC treatment group and conventional treatment group. Table 3 shows the expression relationship table of cytotoxic genes highly expressed in CD14 cell subsets (mono3) in healed patients between MSC treatment group and conventional treatment group. During analysis and processing, it is found that the cell marker genes with high expression of monocyte subsets (especially CD14+ cells) are different, that is, different cell subsets play different functions in the immune response of human body to viral infection. Therefore, in order to treat the complications induced by virus infection, corresponding blocking or activating methods for the signal transduction pathways involved by different cell subsets may be adopted to inhibit cytokine storm in vivo and activate the function of repairing local tissue damage at the same time so as to achieve the therapeutic effects. In other embodiments, patients with other viral infections can also be selected as samples of MSC treatment group and conventional treatment group. It should be noted that when the p_val value in the table is less than 0.01, it means that there is a significant difference between the MSC treatment group and the conventional treatment group, the smaller the data is, the greater the difference is, the p_val_adj value means the value after adjusting the p_val value.

**Table 1 Relationship table of monocyte gene expression in healed patients between MSC treatment group and conventional treatment group**

| Gene name | p_val | avg_logFC | MSC treatment group | conventiona l treatment group | p_val_adj | subset |
|---|---|---|---|---|---|---|
| S100A8 | 1.25E-89 | 0.541755 | 1 | 0.999 | 2.21E-85 | MONO1 |
| S100A9 | 1.39E-17 | 0.339897 | 1 | 1 | 2.44E-13 | MONO2 |
| VCAN | 9.52E-50 | 0.643959 | 0.94 | 0.815 | 1.68E-45 | MONO2 |

It can be seen from Table 1 that the gene expressions of S100A8 gene, S100A9 gene and VCAN gene in monocyte subsets are significantly different between MSC treatment group and conventional treatment group, compared with conventional treatment group, the gene expressions of S100A8 gene, S100A9 gene and VCAN gene in healed patients of MSC treatment group are significantly up-regulated, S 100A8 gene and S 100A9 gene are key molecules in the early stage of cardiac cell death by inhibiting mitochondrial function, and VCAN gene is responsible for coding chondroitinase Versican, which is involved in regulating cell adhesion and migration. At the same time, on the one hand, the mesenchymal stem cells can effectively control the excessive immune response and inhibit the damage to multiple organs of the human body by virtue of low immunogenicity and regulation of congenital and acquired immunity; on the other hand, the mesenchymal stem cells, after entering the human body through intravenous infusion, are partially accumulated in lung, which has the potential to improve the pulmonary cell microenvironment, protect alveolar epithelial cells and improve lung function. Therefore, all clinical studies on mesenchymal stem cells in treating acute lung injury, severe pneumonia, ARDS and respiratory failure have shown that cell infusion has good safety and effectiveness. Therefore, the mesenchymal stem cells exert a regulatory effect by allowing the S 100A8 gene, S 100A9 gene and VCAN gene in Monocyte Subset Mono3 of patients with viral infection to directly contact with target cells or by secreting cytokines, thereby effectively regulating the gene expression of S100A8 gene, S100A9 gene and VCAN gene in Monocyte Subset Mono3.

In summary, the use of mesenchymal stem cells in regulating the gene expression of monocytes in patients with viral infection can effectively inhibit the activation and proliferation of T cells, inhibit the killing function of NK cells, promote the proliferation of regulatory T cells, improve the oxygenation index of healed patients, and reduce the inflammatory injury of the lungs of healed patients, thus realizing the effective treatment of complications induced by patients with viral infection.

**Table 2 Cytotoxic gene table for highly expressed in CD14 cell subsets (mono3) in healed patients between MSC treatment group and conventional treatment group**

| Gene name | p_val | avg_logFC | MSC treatment group | conventional treatment group | p_val_adj | cluster |
|---|---|---|---|---|---|---|
| GNLY | 5.62E-242 | 2.720506 | 0.436 | 0.059 | 9.91E-238 | CD14 mono3 |
| NKG7 | 0 | 2.703494 | 0.665 | 0.168 | 0 | CD14 mono3 |
| GZMB | 8.49E-200 | 1.632217 | 0.429 | 0.021 | 1.50E-195 | CD14 mono3 |
| PRF1 | 4.44E-252 | 1.436399 | 0.499 | 0.016 | 7.83E-248 | CD14 mono3 |

**Table 3 Expression relationship table for cytotoxic genes highly expressed in CD14 cell subsets (mono3) in healed patients between MSC treatment group and conventional treatment group**

| Gene name | p_val | avg_logFC | MSC treatment group | conventional treatment group | p_val_adj |
|---|---|---|---|---|---|
| NKG7 | 0.001507 | -0.33924 | 0.984 | 0.988 | 1 |

It can be seen from Table 2 and Table 3 that the cytotoxic genes GNLY, NKG7, GZMB and PRF1 in CD14 cell subsets (in which mono3 represents the third subset) are highly expressed. Compared with the conventional treatment group, the NKG7 gene of healed patients in MSC treatment group is significantly down-regulated. Because human granulysin expressed by cytotoxic gene GNLY has broad-spectrum cytotoxicity, it has different degrees of killing activity against many bacteria, fungi, parasites and mammalian cells; while the granzyme B encoded by cytotoxic gene GZMB belongs to serine protease family, which is a powerful killer factor in the immune effect of granulosa exocytosis mediated by cytotoxic lymphocytes and can be used as a marker of activation of cytotoxic lymphocytes. Granzyme B is closely related to antiviral infection and participates in many kinds of immune response to intracellular pathogens. Cytotoxic gene PRF1 is responsible for coding perforin 1, which is produced by cells such as cytotoxic T cells, it is usually stored in secretory granules, released when contacted with target cells, and polymerized into tubular pores that can penetrate the target cell membrane to lyse the target cells in the presence of calcium ions. The protein encoded by the cytotoxic gene NKG7 is a kind of cytotoxic protein, granular membrane protein and tetraspanin with molecular weight of 17-kDa, which is located in a kind of lymphocytes with cytolytic ability. As a calcium channel, it is highly expressed in NK cells and activated cytotoxic T cells. At the same time, on the one hand, mesenchymal stem cells can effectively control the excessive immune response and inhibit the damage to multiple organs of the human body by virtue of low immunogenicity and regulation of congenital and acquired immunity; on the other hand, the mesenchymal stem cells, after entering the human body through intravenous infusion, are partially accumulated in lung, which has the potential to improve the pulmonary cell microenvironment, protect alveolar epithelial cells and improve lung function. Therefore, all clinical studies on mesenchymal stem cells in treating acute lung injury, severe pneumonia, ARDS and respiratory failure have shown that cell infusion has good safety and effectiveness. Therefore, the cytotoxic genes GNLY, NKG7, GZMB and PRF1 in the mononuclear cell subset Mono3 of patients with viral infection directly contact with target cells or exert regulatory effect by secreting cytokines through mesenchymal stem cells, thereby effectively regulating the gene expression of toxic genes GNLY, NKG7, GZMB and PRF1.

Thus, the use of mesenchymal stem cells in regulating the gene expression of monocytes in patients with viral infection can effectively inhibit the activation and proliferation of T cells, inhibit the killing function of NK cells, promote the proliferation of regulatory T cells, improve the oxygenation index of healed patients, and reduce the inflammatory injury of the lungs of healed patients, thus realizing the effective treatment of complications induced by patients with viral infection.

The present disclosure also provides the use of mesenchymal stem cells in improving the oxidative stress state of human body, particularly relates to the use of mesenchymal stem cells in improving the oxidative stress state of patients with viral infection, and a single cell sequencing technology is used for detecting and analyzing the expression of antioxidative stress genes of the mesenchymal stem cells, the mesenchymal stem cells are cultured under conventional conditions and oxidative stress stimulation conditions with different concentrations, and then the secretion profile of antioxidative stress factors in mesenchymal stem cells at different times under different conditions is detected and analyzed. The mesenchymal stem cells can highly express multiple antioxidative stress genes and secrete multiple antioxidative stress factors under the stimulation of oxidative stress, which can effectively inhibit the inflammatory reaction and excessive activation of immune cells in the human body (especially patients with viral infection) through antioxidative stress factors. Therefore, mesenchymal stem cells can effectively improve the oxidative stress state of human body.

Wherein the antioxidative stress genes highly expressed in the mesenchymal stem cells include GPX4, GSTP1, PRDX1, PRDX2, PRDX3, PRDX4, PRDX5, PRDX6, SOD1, SOD2, TXNRD1, NQO1, PARK7, PSMB5, SLC38A1 and VIM; the antioxidative stress factors secreted by the mesenchymal stem cells under oxidative stress conditions include GSTP1, PRDX1, PRDX2, VIM, GPX4, PSMB5 and SOD1.

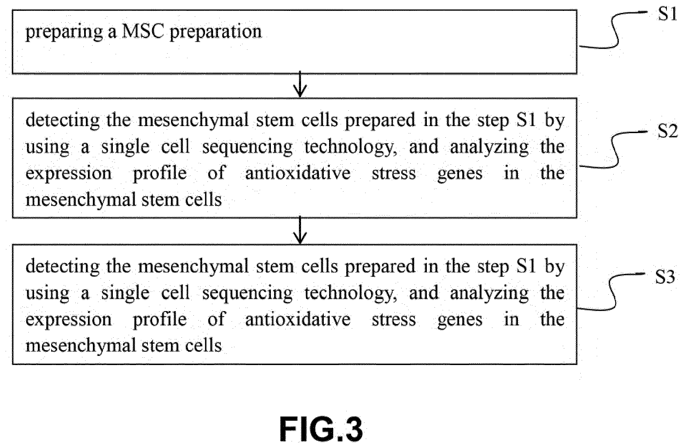

As shown in FIG. 3, the use of the mesenchymal stem cells in improving the oxidative stress state of human body comprises the following steps:
S 1, preparing a mesenchymal stem cell preparation;
S2, detecting the mesenchymal stem cells prepared in the step S1 by using a single cell sequencing technology, and analyzing the expression of antioxidative stress genes in the mesenchymal stem cells;
S3, respectively culturing the mesenchymal stem cells prepared in step S1 under different oxidative stress stimulation conditions, and detecting the secretion profile of antioxidative stress factors in the cultured mesenchymal stem cells.

As shown in FIG. 2, the preparation method of the mesenchymal stem cell preparation comprises:
S11, collecting an umbilical cord and placing the umbilical cord in a culture dish, and then cleaning the umbilical cord tissue with normal saline;
S12, cutting the cleaned umbilical cord tissue into small tissue pieces and planting the small tissue pieces in a culture dish so as to perform the culturing;
S13, removing the culture solution from the culture dish, washing with normal saline, adding pancreatin for digestion until the cells in the culture dish are digested, adding stopping solution to stop digestion, transferring the cell suspension to a centrifuge tube for centrifugation and discarding supernatant, resuspending the cells with an appropriate amount of culture solution and counting, and finally planting the cell suspension in a new culture dish so as to culture the cell suspension according to the counting result;
S14, removing the culture solution from the new culture dish in step S13, washing it with normal saline, adding pancreatin for digestion and adding stop solution to stop digestion until the cells in the culture dish are digested, filtering with a cell sieve, transferring the filtered cell suspension to a centrifuge tube, counting and centrifuging to discard supernatant, preparing a cell preparation suspension, adding the cell preparation suspension to resuspend the cells, and finally transferring the cell suspension to a transfer bag and placing the transfer bag in a low temperature environment for subsequent withdrawal, thus completing the preparation of mesenchymal stem cell preparation;
S15, qualifying the mesenchymal stem cell preparation prepared in step S14.

In the example, the mesenchymal stem cell preparation are prepared by using human umbilical cord mesenchymal stem cells (hUC-MSC), and the preparation process is carried out in a sterilized clean bench, and the instruments and consumable supplies required in the preparation process need to be disinfected with 75% alcohol, the collected objects or cells in the culture dish and the specific operation process need to be marked in detail during the preparation process. At the same time, the stem cells can be frozen between step S13 and step S14. If it is necessary to perform step S14, the cryopreserved stem cells need to be resuscitated before preparing the stem cells preparation in step S14, the freezing and resuscitation operations of stem cells is the prior art, and they will not be described in detail here.

In the example, the quality detection of the mesenchymal stem cell preparation also includes collection quality detection, admittance detection of umbilical cord mesenchymal stem cells in the primary cell bank and admittance detection of umbilical cord mesenchymal stem cells in master cell bank, and the steps and standards for the quality detection are consistent with the existing common detection procedure, and they will not be described in detail here.

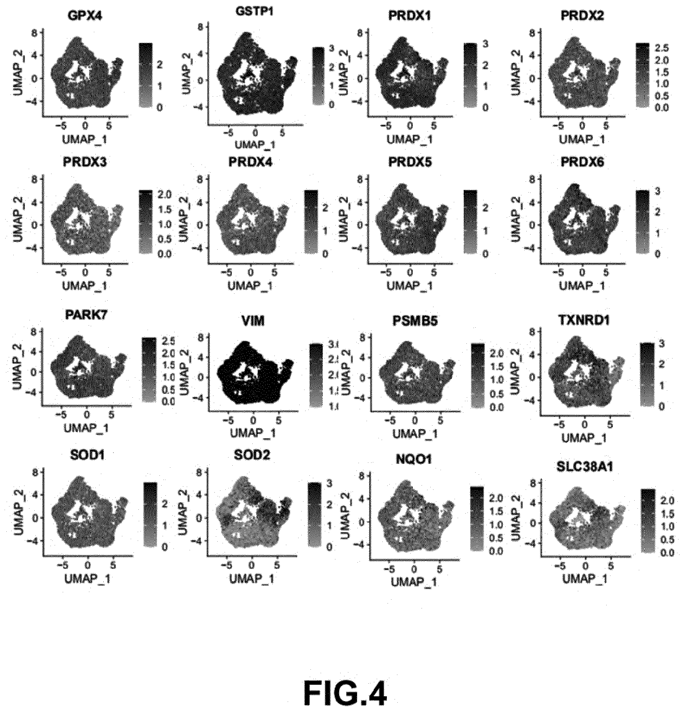

As shown in FIG. 4, the specific implementation of step S2 includes detecting the prepared mesenchymal stem cells by using a single cell sequencing technology, and analyzing the expression profile of antioxidant stress genes in the mesenchymal stem cells.

The specific implementation mode of step S3 includes: firstly, culturing the prepared mesenchymal stem cells under conventional conditions and oxidative stress stimulation conditions with different concentrations, and then detecting and analyzing the secretion profile of antioxidative stress factors in the mesenchymal stem cells under different conditions and in different periods.

In this example, it can be seen from FIG. 4 that the single cell sequencing technology is used to detect the prepared mesenchymal stem cells and analyze the expression of antioxidative stress genes in the mesenchymal stem cells, the antioxidative stress genes highly expressed in the mesenchymal stem cells include GPX4, GSTP1, PRDX1, PRDX2, PRDX3, PRDX4, PRDX5, PRDX6, SOD1, SOD2, TXNRD1, NQO1, PARK7, PSMB5, SLC38A1 and VIM. In addition, the mesenchymal stem cells are cultured under conventional conditions and oxidative stress stimulation conditions with different concentrations, and the secretion profile of antioxidative stress factors in mesenchymal stem cells at different times under different conditions is detected and analyzed, the test results show that mesenchymal stem cells can secrete multiple antioxidative stress factors under the stimulation of different concentrations of hydrogen peroxide, including GSTP1, PRDX1, PRDX2, VIM, GPX4, PSMB5 and SOD1, and all of these antioxidative stress factors exert a good antioxidative effect, and therefore, they can have a positive effect on improving the oxidative stress state of human body. In this example, the supernatant of cultured mesenchymal stem cells is detected and analyzed, and the detection results are shown in Table 4. Table 4 shows the secretion profile of antioxidative stress factors after mesenchymal stem cells are cultured under conventional conditions and oxidative stress stimulation conditions with different concentrations.

**Table 4 Secretion profile of antioxidative stress factors after mesenchymal stem cells are cultured under conventional conditions and oxidative stress stimulation conditions with different concentrations**

| Antioxidative stress factors (ng/mL) | | GSTP1 | PRDX1 | PRDX2 | VIM | GPX4 | PSMB5 | SOD1 |
|---|---|---|---|---|---|---|---|---|
| H₂O₂ (0µM) | 6h | 212.96 | 0.00 | 0.056 | 195.47 | 9.18 | 290.92 | 13744.35 |
| | 12h | 268.21 | 0.22 | 0.058 | 280.70 | 9.88 | 277.95 | 15009.58 |
| | 24h | 433.73 | 0.00 | 0.059 | 493.27 | 10.43 | 298.41 | 15754.44 |
| H₂O₂ (50µM) | 6h | 383.95 | 0.13 | 0.054 | 204.49 | 8.70 | 293.27 | 39242.89 |
| | 12h | 276.98 | 0.65 | 0.055 | 413.06 | 14.37 | 272.79 | 29672.01 |
| | 24h | 372.90 | 0.86 | 0.069 | 1063.81 | 9.55 | 304.47 | 26008.95 |
| H₂O₂ (100µM) | 6h | 216.26 | 0.39 | 0.056 | 211.51 | 10.32 | 277.61 | 12448.50 |
| | 12h | 327.90 | 0.34 | 0.054 | 279.70 | 10.53 | 284.67 | 15019.78 |
| | 24h | 772.06 | 0.44 | 0.062 | 590.53 | 10.76 | 328.02 | 35834.92 |
| H₂O₂ (200µM) | 6h | 200.20 | 0.63 | 0.056 | 174.41 | 9.86 | 283.24 | 19886.85 |
| | 12h | 353.99 | 1.00 | 0.06 | 347.88 | 10.59 | 298.75 | 17233.94 |
| | 24h | 607.11 | 0.71 | 0.084 | 465.20 | 8.43 | 332.15 | 23080.55 |

It can be seen from Table 4 that compared with mesenchymal stem cells cultured under conventional conditions (i.e., the concentration of hydrogen peroxide is 0 µM), after external oxidative stress stimulation (i.e., stimulation with hydrogen peroxide concentration of 50 µM, 100 µM and 200 µM, respectively), multiple antioxidative stress factors in mesenchymal stem cells increase in different periods (i.e., 6 h, 12 h and 24 h after mesenchymal stem cells are cultured). Therefore, mesenchymal stem cells can secrete multiple antioxidative stress factors, mesenchymal stem cells can effectively improve the oxidative stress state of the human body.

In order to further explain the working principle and technical effect of the present disclosure, the patients with normal neocoronal positive (i.e., patients with viral infection of SARS-CoV-2) are divided into two groups and use them as samples of the MSC treatment group and the conventional treatment group (i.e., the original treatment plan), wherein the treatment regimen of the MSC treatment group is to incorporate the prepared mesenchymal stem cell preparation into the MSC treatment group and use the mesenchymal stem cell preparation as the only interference factor in the conventional treatment regimen to carry out combined medication for patients with viral infection. The treatment regimen of the conventional treatment group refers to the original treatment regimen, that is, the treatment regimen adopts in the diagnosis and treatment regimen of novel coronavirus by the Office of the National Health Commission and Office of the State Administration of Traditional Chinese Medicine. The results show that compared with the conventional treatment group, the oxidative stress state of the healed patients in the MSC treatment group can be effectively improved. Therefore, it is concluded that the combined treatment by using mesenchymal stem cell preparation in the MSC treatment group can effectively inhibit the the inflammatory reaction and excessive activation of immune cells in the healed patients with the viral infection, and to enable improvement of inflammatory indexes and myocardial injury indexes of human body. Thus, it is concluded that mesenchymal stem cells can improve the oxidative stress state of the human body, especially the oxidative stress state of patients with viral infection, whereas mesenchymal stem cells inhibit the inflammatory reaction and excessive activation of immune cells in the healed patients with the viral infection by high expression of multiple antioxidative stress genes and secretion of multiple antioxidative stress factors.

Therefore, the mesenchymal stem cell preparation can highly express multiple antioxidative stress genes and secrete multiple antioxidative stress factors in oxidative damage environment, thereby effectively improving the oxidative stress state of human body.

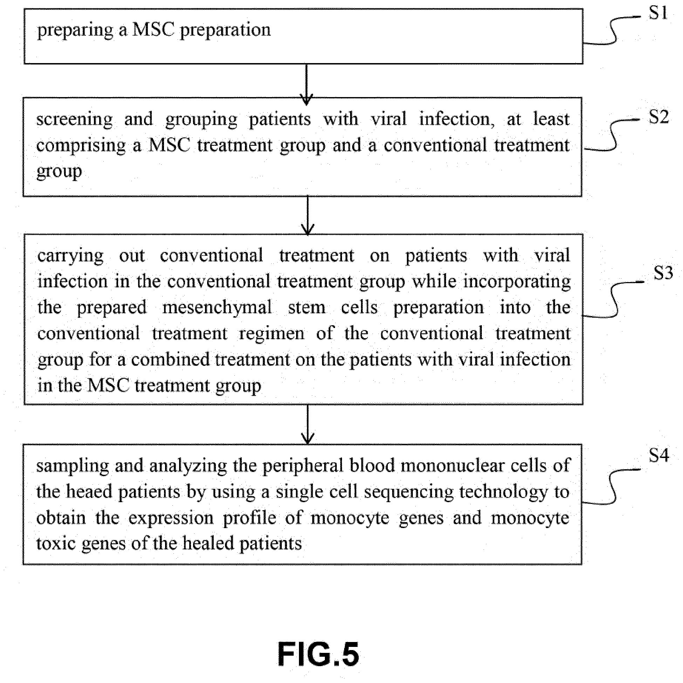

As shown in FIG. 5, the present disclosure provides the use of MSC in regulating the gene expression of terminally differentiated cells of patients with viral infection, and realizes effective treatment of a complication induced by viral infection by using MSC to regulate the expression of antivirus-related genes and coagulation-related genes of terminally differentiated cell subsets of patients. Wherein, the complications induced by viral infection is acute respiratory distress syndrome, and the specific use comprises steps of:
S 1, preparing a MSC preparation;
S2, screening and grouping patients with viral infection, at least comprising a MSC treatment group and a conventional treatment group;
S3, carrying out conventional treatment on patients with viral infection in the conventional treatment group while incorporating the prepared MSC preparations into the MSC treatment group so as to impose a combined treatment on patients with viral infection in the MSC treatment group;
S4, sampling and analyzing peripheral blood mononuclear cells of the healed patients by using a single cell sequencing technology to obtain the expression profile of antivirus-related genes and coagulation-related genes of terminally differentiated cell subsets of the healed patients.

As shown in FIG. 2, the preparation method of the MSC preparations comprises:
S11, collecting an umbilical cord and placing the umbilical cord in a culture dish, and then cleaning the umbilical cord tissue with normal saline;
S12, cutting the cleaned umbilical cord tissue into small tissue pieces and planting the small tissue pieces in a culture dish so as to perform the culturing;
S13, removing the culture solution from the culture dish, washing with normal saline, adding pancreatin for digestion until the cells in the culture dish are digested, adding stopping solution to stop digestion, transferring the cell suspension to a centrifuge tube for centrifugation and discarding supernatant, resuspending the cells with an appropriate amount of culture solution and counting, and finally planting the cell suspension in a new culture dish so as to culture the cell suspension according to the counting result;
S14, removing the culture solution from the new culture dish in step S13, washing it with normal saline, adding pancreatin for digestion and adding stop solution to stop digestion until the cells in the culture dish are digested, filtering with a cell sieve, transferring the filtered cell suspension to a centrifuge tube, counting and centrifuging to discard supernatant, preparing a cell preparation suspension, adding the cell preparation suspension to resuspend the cells, and finally transferring the cell suspension to a transfer bag and placing the transfer bag in a low temperature environment for subsequent withdrawal, thus completing the preparation of MSC preparations;
S15, qualifying the MSC preparation prepared in step S14.

In the example, the MSC preparation is prepared by using human umbilical cord mesenchymal stem cells (hUC-MSC), and the preparation process is carried out in a sterilized clean bench, and the instruments and consumable supplies required in the preparation process need to be disinfected with 75% alcohol, the collected materials or cells in the culture dish and the specific operation process need to be marked in detail during the preparation process. At the same time, the stem cells can be frozen between step S13 and step S14. If it is necessary to perform step S14, the cryopreserved stem cells need to be resuscitated before preparing the stem cells preparation in step S14, the freezing and resuscitation operations of stem cells is an prior art, and they will not be described in detail here.

In the example, the quality detection of the MSC preparations also includes collection quality detection, admittance detection of umbilical cord mesenchymal stem cells in the primary cell bank and admittance detection of umbilical cord mesenchymal stem cells in master cell bank, and the steps and standards for the quality detection are consistent with the existing common detection procedure, and they will not be described in detail here.

Wherein, the combined treatment in step S3 refers to a treatment in which the MSC preparations prepared in step S1 are incorporated into the combined treatment regimen in the MSC treatment group, and the MSC preparations are used as the only interference factor in the original conventional treatment regimen to carry out combined medication for patients with viral infection, and the combined medication situation is tracked and recorded in the combined treatment regimen. In the example, the MSC preparations are infused through peripheral vein during the whole MSC treatment group, and the infusion dose of the mesenchymal stem cell preparation is (0.8-1)×10⁶ cells per kilogram of body weight, the infusions is conducted for 3 times, and the interval between every two infusions is 3 days, the infusion rate is 10 drops/ min from 0 to 15 min, and the infusion is continued at a rate of 20 drops/ min if there is no significant response. The number of monocytes in patients with viral infection is more than that in healthy individuals, especially CD14+ monocyte subsets (CD14+mono4 and CD14+mono5, namely the fourth and the fifth subsets of CD14 cells) are hardly found in healthy individuals, whereas the genes IFIT1, IFIT2, IFIT3 and ISG15 in CD14+mono5 cell subsets are mainly involved in the process of virus defense, and are responsible for regulating the replication of virus genome and cellular response to interferon α, meanwhile, the gene PPBP, gene PF4, gene TREML1 and gene PF4V1 in CD14+mono4 cell subsets are mainly involved in the viral defense process, as well as being responsible for regulating the replication of the viral genome and the cellular response to interferon α, while gene PPBP, gene PF4, gene treml1, and gene pf4v1 in CD14 + mono4 cell subset are mainly involved in the platelet threshing process, and positively regulate leukocyte chemotaxis and response to lipopolysaccharide. It can be seen that during the period of infection and the development of complications in patients with viral infection, the subsets CD14 + mono5 and CD14 + mono5 play a key role in antivirus, antiinfection and coagulation.

In this example, the selected patients with normal novel coronal positive (i.e., patients with viral infection of SARS-CoV-2) are divided into two groups and use them as samples of the MSC treatment group and the conventional treatment group (i.e., the original treatment plan). The peripheral blood mononuclear cells of the healed patients are sampled after the patients with viral infection in the both groups are cured. The peripheral blood mononuclear cells of the healed patients (that is, cell samples from patients with viral infectious complications) are analyzed in respective groups by using the single cell sequencing technology and the expression levels of antivirus-related genes and coagulation function related genes in MSC treatment group and conventional treatment group are obtained, the details are as follows: Table 5 shows the expression levels of antivirus-related genes of monocytes in MSC treatment group and conventional treatment group. Table 6 shows the type I interferon signal pathways and antivirus related gene expression levels in MSC treatment group and conventional treatment group that are the most effective against viruses. Table 7 shows the expression levels of coagulation function related gene in MSC treatment group and conventional treatment group. Wherein, it should be noted that when the p value in the table is less than 0.01, it means that there is a significant difference between the MSC treatment group and the conventional treatment group, the smaller the data is, the greater the difference is.

**Table 5 Expression levels of antivirus-related genes of monocytes in MSC treatment group and conventional treatment group**

| Gene name | *p* value | avg_logFC | MSC treatment group | conventional treatment group |
|---|---|---|---|---|
| IFIT 1 | 2.43E-166 | 1.2812338 | 0.674 | 0.092 |
| IFIT2 | 1.40E-138 | 1.25618168 | 0.766 | 0.185 |
| IFIT3 | 1.81E-160 | 1.23330438 | 0.805 | 0.199 |
| ISG15 | 2.45E-250 | 1.64037884 | 0.974 | 0.485 |

**Table 6 The type I interferon signal pathways and antivirus related gene expression levels in MSC treatment group and conventional treatment group**

| Gene name | *p* value | avg_logFC | MSC group treatment | conventional treatment group |
|---|---|---|---|---|
| IFITM1 | 2E-12 | -0.847847876 | 0.1 | 0.452 |
| IFITM2 | 5.28E-13 | -0.677681018 | 0.875 | 0.948 |
| IFITM3 | 3.09E-38 | -1.28873318 | 0.842 | 0.984 |
| ISG15 | 3.96E-20 | -1.325335035 | 0.267 | 0.706 |

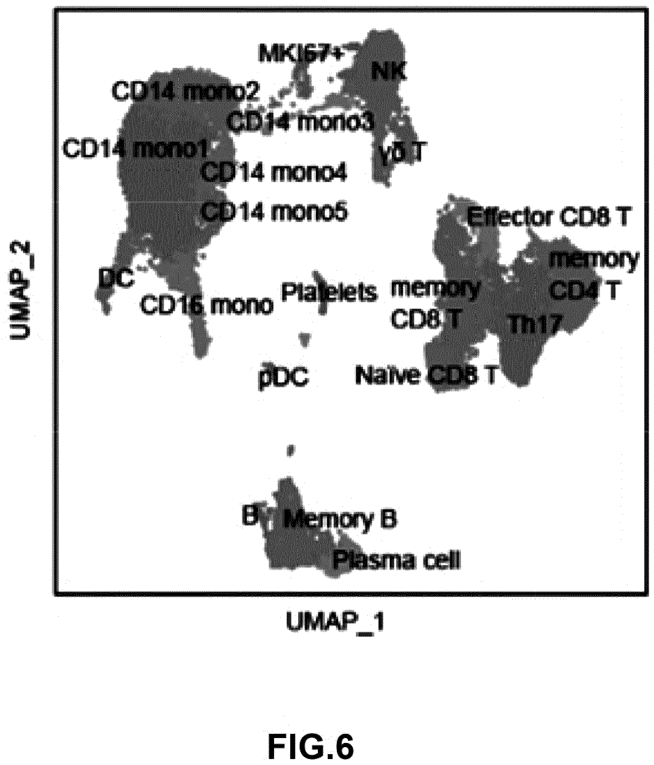

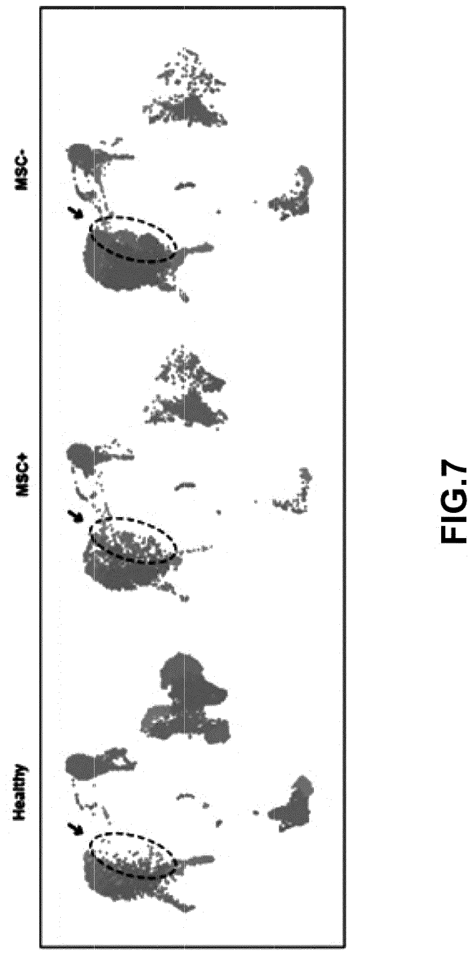

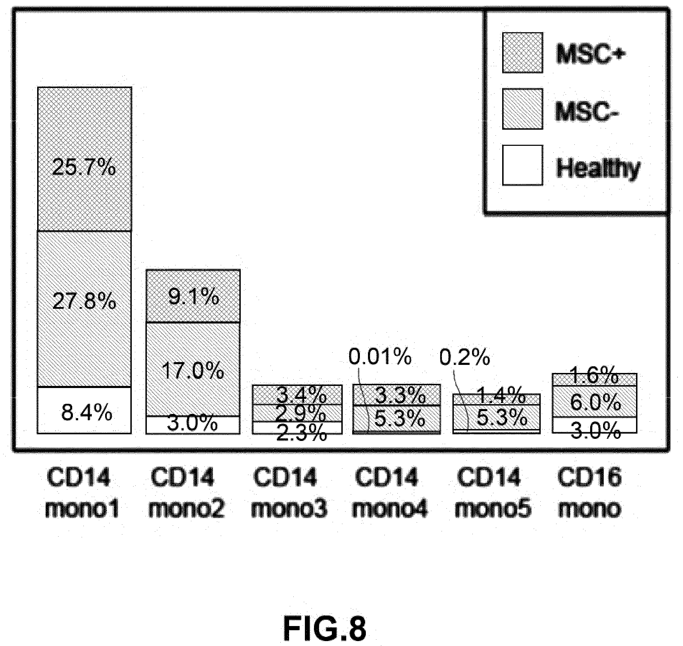

It can be seen from Table 5, Table 6, FIG. 6, FIG. 7 and FIG. 8, that CD14+mono5 cell subsets are hardly found in healthy individuals, which indicates that CD14+mono5 cell subsets in monocytes only exist in patients with viral infection, and the proportion of CD14+mono5 cell subsets in MSC treatment group is 1.4%, while the proportion of CD14+mono5 cells in the conventional treatment group is 6.0%. Compared with the conventional treatment group, the gene expression level of a series of genes (such as gene IFIT1, gene IFIT2, gene IFIT3 and gene ISG15) involved in type I interferon signal pathway and antiviral response in MSC treatment group is significantly lower, and the gene expression level of CD14+mono5 cell subsets in MSC treatment group is also lower.

**Table 7 Expression levels of coagulation function related gene in MSC treatment group and conventional treatment group**

| Genes | *p* value | avg_logFC | MSC treatment group | conventional treatment group |
|---|---|---|---|---|
| PPBP | 0.00E+00 | 2.49383929 | 0.984 | 0.329 |
| PF4 | 1.61E-264 | 1.66379327 | 0.863 | 0.17 |
| TREML1 | 8.88E-210 | 1.15783275 | 0.627 | 0.048 |
| PF4V1 | 2.09E-158 | 1.19303336 | 0.611 | 0.095 |

It can be seen from Table 6, Table 7, FIG. 6, FIG. 7 and FIG. 8, that CD14+mono4 cell subsets are hardly found in healthy individuals, which indicates that CD14+mono4 cell subsets in monocytes only exist in patients with viral infection, and the proportion of CD14+mono4 cell subsets in MSC treatment group is 3.3%, while the proportion of CD14+mono4 cells in the conventional treatment group is 5.3%. Compared with the conventional treatment group, the gene expression level of genes involved in type I interferon signal pathway in MSC treatment group is significantly lower, and the gene expression level of CD14+mono5 cell subsets in MSC treatment group is also lower.

In summary, MSC can inhibit the excessive activation of monocytes in patients with viral infection, which is very beneficial to the recovery of monocytes from excessive activation, MSC can effectively inhibit the number of activated monocytes in patients with viral infection (including the expression of antivirus-related genes mono5 and the expression of coagulation-related genes mono4), and regulate the gene expression level of inflammatory genes. Therefore, regulating the gene expression of terminally differentiated cells in patients with viral infection by MSC can improve the clinical symptoms of patients with viral infection, greatly reduce the mortality of patients, and can be an effective treatment for complications induced by viral infection.

It should be noted that in Table 5, Table 6 and Table 7, when avg-logFC value is positive, it is indicated that the expression of the gene in MSC treatment group is up-regulated compared with conventional treatment group; when avg-logFC value is negative, it is indicated that the expression of the gene in MSC treatment group is down-regulated compared with conventional treatment group; when p value is less than 0.05, it is indicated that the expression of the gene is significantly different between MSC treatment group and conventional treatment group.

The above described are only preferred embodiments of the present disclosure, it should be noted by those skilled in the art that, several improvements and modifications can be made without departing from the principle of the present disclosure, and these improvements and modifications also should be regarded as the protection scope of the present application and fall into the scope of the present application.

## Claims

1. Mesenchymal stem cells for use in regulating monocytes in patients with viral infection, regulating gene expression of terminally differentiated cells in patients with viral infection, or improving oxidative stress state of human body.

2. The mesenchymal stem cells according to claim 1, wherein the mesenchymal stem cells are used for regulating gene expression and/or toxic gene expression of monocytes in patients with viral infection to achieve effective treatment of a complication induced by viral infection when the mesenchymal stem cells are used for regulating monocytes in patients with viral infection;
the mesenchymal stem cells are used for regulating the expression of antivirus-related genes and/or coagulation-related genes of terminally differentiated cell subsets in patients with viral infection to achieve effective treatment of a complication induced by viral infection when the mesenchymal stem cells are used for regulating the gene expression of terminally differentiated cells in patients with viral infection;
the mesenchymal stem cells are used for improving the oxidative stress state of patients with viral infection when the mesenchymal stem cells are used for improving oxidative stress state of human body.

3. The mesenchymal stem cells according to claim 2, wherein the mesenchymal stem cells are used for improving the oxidative stress state of patients with viral infection by highly expressing multiple antioxidative stress genes and secreting multiple antioxidative stress factors.

4. The mesenchymal stem cells according to claim 3, wherein the antioxidative stress genes highly expressed in the mesenchymal stem cells comprise GPX4, GSTP1, PRDX1, PRDX2, PRDX3, PRDX4, PRDX5, PRDX6, SOD1, SOD2, TXNRD1, NQO1, PARK7, PSMB5, SLC38A1 and VIM.

5. The mesenchymal stem cells according to claim 3, wherein the antioxidative stress factors secreted by the mesenchymal stem cells under oxidative stress conditions comprise GSTP1, PRDX1, PRDX2, VIM, GPX4, PSMB5 and SOD1.

6. The mesenchymal stem cellse according to claim 2, wherein the complication induced by viral infection is acute respiratory distress syndrome.

7. The mesenchymal stem cells according to claim 6, wherein when the mesenchymal stem cells are used for regulating monocytes in patients with viral infection, the use comprising steps of:
S1, preparing a mesenchymal stem cell preparation;
S2, screening and grouping patients with viral infection, at least comprising a mesenchymal stem cell treatment group and a conventional treatment group;
S3, carrying out conventional treatment on patients with viral infection in the conventional treatment group while incorporating the prepared mesenchymal stem cell preparation into the conventional treatment regimen of the conventional treatment group so as to impose a combined treatment on the patients with viral infection in the mesenchymal stem cell treatment group;
S4, sampling and analyzing the peripheral blood mononuclear cells of healed patients by using a single cell sequencing technology to obtain the expression profile of monocyte genes and monocyte toxic genes of the healed patients.

8. The mesenchymal stem cells according to claim 6, wherein when the mesenchymal stem cells are used for regulating gene expression of terminally differentiated cells in patients with viral infection, the use comprising steps of:
S1, preparing a mesenchymal stem cell preparation;
S2, screening and grouping patients with viral infection, at least comprising a mesenchymal stem cell treatment group and a conventional treatment group;
S3, carrying out conventional treatment on patients with viral infection in the conventional treatment group while incorporating the prepared mesenchymal stem cell preparation into the conventional treatment regimen of the conventional treatment group so as to impose a combined treatment on the patients with viral infection in the mesenchymal stem cell treatment group;
S4, sampling and analyzing peripheral blood mononuclear cells of the healed patients by using a single cell sequencing technology to obtain the expression profile of antivirus-related genes and coagulation-related genes of terminally differentiated cell subsets of the healed patients.

9. The mesenchymal stem cells according to claim 7 or 8, wherein the healed patients comprise patients with viral infection cured in mesenchymal stem cell treatment group and patients with viral infection cured in conventional treatment group.

10. The mesenchymal stem cells according to claim 5, wherein when the mesenchymal stem cells are used for improving the oxidative stress state of human body, the use comprising steps of:
S1, preparing a mesenchymal stem cell preparation;
S2, detecting the mesenchymal stem cells prepared in step S1 by using a single cell sequencing technology, and analyzing the expression profile of antioxidant stress genes in the mesenchymal stem cells;
S3, respectively culturing the mesenchymal stem cells prepared in step S1 under different oxidative stress stimulation conditions, and detecting the secretion profile of antioxidative stress factors in the cultured mesenchymal stem cells.

11. The mesenchymal stem cells according to claim 10, wherein the specific implementation of step S2 comprises:
detecting the prepared mesenchymal stem cells by using a single cell sequencing technology, and analyzing the expression profile of multiple antioxidative stress genes of the mesenchymal stem cells.

12. The mesenchymal stem cells according to claim 10, wherein the specific implementation of step S3 comprises: culturing the prepared mesenchymal stem cells under conventional conditions and oxidative stress stimulation conditions with different concentrations, and then detecting and analyzing the secretion profile of antioxidative stress factors in the mesenchymal stem cells under different conditions and in different periods.

13. The mesenchymal stem cells according to any one of claims 7, 8 and 10, wherein the preparation method of the mesenchymal stem cell preparation comprise:
S11, collecting an umbilical cord and placing the umbilical cord in a culture dish, and then cleaning the umbilical cord tissue with normal saline;
S12, cutting the cleaned umbilical cord tissue into small tissue pieces and planting the small tissue pieces in a culture dish so as to perform the culturing;
S13, removing the culture solution from the culture dish, washing with normal saline, adding pancreatin for digestion until the cells in the culture dish are digested, adding stopping solution to stop digestion, transferring the cell suspension to a centrifuge tube for centrifugation and discarding supernatant, resuspending the cells with an appropriate amount of culture solution and counting, and finally planting the cell suspension in a new culture dish so as to culture the cell suspension according to the counting result;
S14, removing the culture solution from the new culture dish in step S13, washing it with normal saline, adding pancreatin for digestion and adding stop solution to stop digestion until the cells in the culture dish are digested, filtering with a cell sieve, transferring the filtered cell suspension to a centrifuge tube, counting and centrifuging to discard supernatant, preparing a cell preparation suspension, adding the cell preparation suspension to resuspend the cells, and finally transferring the cell suspension to a transfer bag and placing the transfer bag in a low temperature environment for subsequent withdrawal, thus completing the preparation of mesenchymal stem cell preparation;
S15, qualifying the mesenchymal stem cell preparation prepared in step S14.

14. Mesenchymal stem cells for use in regulating gene expression of terminal differentiation cells and monocyte genes of patients with viral infection or improving oxidative stress state of human body, comprising the following steps:
1) preparing a mesenchymal stem cell preparation;
2) incorporating the mesenchymal stem cell preparation into conventional treatment regimen so as to impose a combined treatment of patients with viral infection.
